# EUROPÄISCHE PATENTSCHRIFT

(11) **EP 1 103 537 B1**
(45) Veröffentlichungstag und Bekanntmachung des Hinweises auf die Patenterteilung: **14.05.2003**
(21) Anmeldenummer: 00811058.7
(22) Anmeldetag: 10.11.2000
(51) Int. Cl.: C07C 45/30, C07F 9/6581, C07F 9/59, C08G 79/00

(54) **Verfahren zur selektiven Oxidation von Alkoholen unter Verwendung leicht abtrennbarer Nitroxylradikale**
Process for the selective oxidation of alcohols using easily separable nitroxyl radicals
Procédé pour l'oxydation sélective d'alcools utilisant des radicaux nitroxyles facilement séparables

(30) Priorität: 19.11.1999 CH 211399
(43) Veröffentlichungstag der Anmeldung: 30.05.2001
(73) Patentinhaber: Ciba Specialty Chemicals Holding Inc., 4057 Basel (CH)
(72) Erfinder: Sommerlade, Reinhard, 79112 Freiburg (DE); Grützmacher, Hansjörg, 8907 Wettswil (CH); Boulmaâz, Souâd, 4127 Birsfelden (CH)

(56) Entgegenhaltungen:
- WO-A-99/29646
- NOOY DE A E J ET AL: "ON THE USE OF STABLE ORGANIC NITROXYL RADICALS FOR THE OXIDATION OFPRIMARY AND SECONDARY ALCOHOLS" SYNTHESIS,DE,GEORG THIEME VERLAG. STUTTGART, 1. Oktober 1996 (1996-10-01), Seiten 1153-1174, XP002072173 ISSN: 0039-7881
- RYCHNOVSKY S D ET AL: "TEMPO-CATALYZED OXIDATIONS OF ALCOHOLS USING M-CPBA: THE ROLE OF HALIDE IONS" JOURNAL OF ORGANIC CHEMISTRY,US,AMERICAN CHEMICAL SOCIETY. EASTON, Bd. 64, Nr. 1, 8. Januar 1999 (1999-01-08), Seiten 310-312, XP000789492 ISSN: 0022-3263
- CHEMICAL ABSTRACTS, vol. 120, no. 19, 9. Mai 1994 (1994-05-09) Columbus, Ohio, US; abstract no. 244345, KYOSHIMA, JUJIRO: "Preparation of m-phenoxybenzaldehyde as insecticides material" XP002162250 & JP 05 310632 A (SUMITOMO CHEMICAL CO, JAPAN) 22. November 1993 (1993-11-22)
- B HINZEN ET AL: "Polymer supported perruthenate (PSP): Clean oxidation of primary alcohols to carbonyl compounds using oxygen as cooxidant" SYNTHESIS,GEORG THIEME VERLAG. STUTTGART,DE, 1998, Seiten 977-979, XP002156866 ISSN: 0039-7881
- DULOG L ET AL: "A 2 5-CYLOTRIPHOSPHAZENE WITH NITROXYL GROUPS AS MODEL FOR A PARAMAGNETIC POLY(ORGANO-2 5-PHOSPHAZENE)" MAKROMOLEKULARE CHEMIE, MACROMOLECULAR CHEMISTRY AND PHYSICS,CH,HUTHIG UND WEPF VERLAG, BASEL, Bd. 189, Nr. 11, 1. November 1988 (1988-11-01), Seiten 2611-2615, XP000020438 ISSN: 0025-116X

## Beschreibung

Die Erfindung betrifft ein Verfahren zur selektiven Oxidation von Alkoholen zu Ketonen oder Aldehyden mit einem leicht abtrennbaren, heterogenen Oxidationskatalysator auf Basis eines Nitroxylradikals, unter Verwendung eines Alkalihypohalogenits als Oxidationsmittel.

Alkohole zählen zu den wichtigsten organischen Synthesebausteinen überhaupt. Ein umfangreiches präparatives Methodenarsenal zu ihrer Herstellung machen primäre und sekundäre Alkohole zu idealen Vorstufen für die Synthese von Aldehyden, Ketonen und Carbonsäuren. Gebräuchliche Oxidationsmittel sind Schwermetallreagentien wie z. B. Chrom-(VI)-, Blei-(IV)-, und Ruthenium-, Mangan- und Vanadium-Verbindungen, Persäuren, aktiviertes Dimethylsulfoxid (DMSO) und hypervalente Iod-Verbindungen.

Der Selektivität kommt in diesen Oxidationen eine überragende Bedeutung zu. Weitere im Molekül vorhandene funktionelle Gruppen, wie z. B. Doppelbindungen, sollen in der Regel unter den gewählten Bedingungen nicht angegriffen werden. Oftmals ist auch die gezielte Oxidation sekundärer neben primären Alkoholfunktionen und umgekehrt erwünscht, ohne die jeweils andere Funktion anzugreifen. Bei der Synthese von Aldehyden aus primären Alkoholen entstehen oft Carbonsäuren als Nebenprodukte der Oxidationsreaktion (Überoxidation), die Oxidation von 1,2-Diolen oder α-Hydroxyketonen geht häufig mit C-C-Spaltungsreaktionen einher.
Ein weiterer Nachteil vieler Oxidantien besteht in deren oft umständlicher oder schwieriger Herstellung oder Handhabung; darüber hinaus besitzen vor allem schwermetallhaltige Reagentien zumeist eine hohe Toxizität und Umweltschädlichkeit. Schliesslich aber spielen vor allem für den industriellen Einsatzzweck die Kosten eines Oxidationsverfahrens eine entscheidende Rolle.

Es ist bekannt, dass sich primäre und sekundäre Alkohole in Gegenwart katalytischer Mengen an organischen Nitroxyl-Radikalen mit wässriger Natriumhypochloritlösung in die entsprechenden Carbonylverbindungen überführen lassen (A. E. J. de Nooy, A. C. Besemer, H. van Bekkum, *Synthesis*, 1996, 1153).

Bislang wurden diese Umsetzungen - insbesondere unter Verwendung von 2,2,6,6-Tetramethylpiperidin-1-oxyl (TEMPO) - vorwiegend in homogener Phase durchgeführt. Die Reaktionen wurden entweder stöchiometrisch oder katalytisch bezüglich TEMPO oder dessen daraus resultierendem Oxidationsprodukt durchgeführt. Die Aufarbeitung der Reaktionsprodukte gestaltet sich dabei oft umständlich, da der Katalysator und dessen Begleitprodukte mühsam abgetrennt werden müssen.

Oxidationen mit immobilisierten oder leicht abtrennbaren Nitroxylverbindungen dagegen sind bisher nicht beschrieben worden.

Es wurde nun überraschend gefunden, dass Alkohole durch Verwendung von bestimmten höhermolekularen bzw. oligomeren oder polymerfixierten 2,2,6,6-Piperidin-1-oxylen als Katalysator, mit Natriumhypochlorit als Oxidans in guten Ausbeuten zu den entsprechenden Carbonylverbindungen umgesetzt werden können.

Aliphatische 1,3-Diole lassen sich in Gegenwart eines Aldehyds oder Ketons unter geeigneten Versuchsbedingungen in basischem Milieu direkt zu den entsprechenden cyclischen Acetalen bzw. Ketalen (1,3-Dioxanen) umsetzen. 1,5-Diole werden je nach Versuchsbedingungen zu Tetrahydropyran-2-olen bzw. deren Ethern oder zu Tetrahydropyran-2-onen (δ-Valerolactonen) umgesetzt. Hydroxyfunktionen in α-Stellung zu Carboxyfunktionen werden nicht angegriffen.

Ein Zusatz von Bromid, der bei homogen durchgeführter Oxidation mit dem System Hypochlorit / TEMPO zu einer erheblichen Reaktionsbeschleunigung führt (S. D. Rychnovsky, R. Vaidyanathan, *J. Org. Chem*., 1999, *64,* 310), kann in diesem Verfahren ohne Nachteile entfallen. Die Vorzüge des vorliegenden Verfahrens bestehen in der vereinfachten Aufarbeitung der Reaktionsansätze, der mehrfachen Wiederverwendung des Katalysators und im Verzicht von Bromid als reaktionsbeschleunigendem Zusatz.

Ein Gegenstand der Erfindung ist ein Verfahren zur selektiven Oxidation von Alkoholen zu Ketonen oder Aldehyden mit einem Alkalihypohalogenit unter alkalischen Bedingungen, dadurch gekennzeichnet, dass man die Oxidation in Gegenwart eines im Reaktionsmedium unlöslichen heterogenen Oxidationskatalysators, ausgewählt aus der Gruppe enthaltend die Verbindungen
der Formel (I), (II), (III) 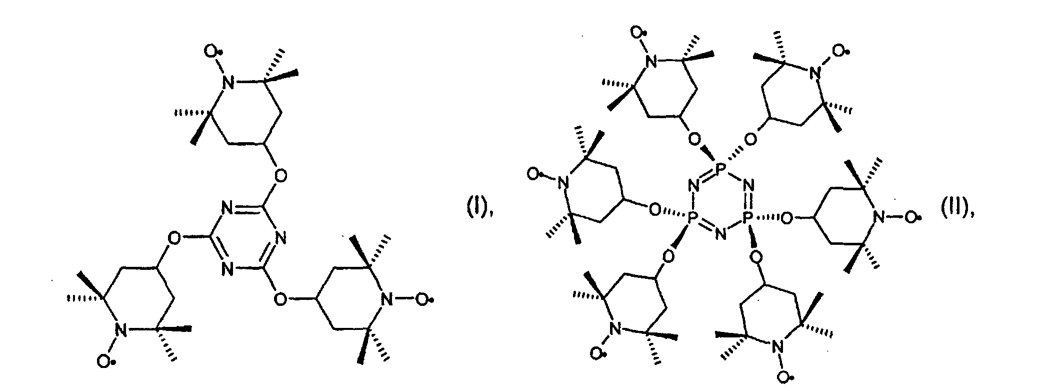 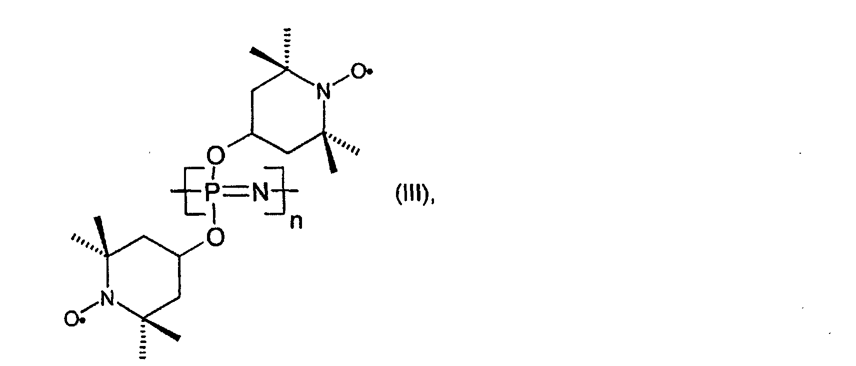 worin n eine Zahl von 3-3000ist; oder
ein an ein Merrifield-Polymer 4-oxy gebundenes 4-oxy-2,2,6,6-tetramethylpiperidin-1-oxyl, durchführt.

Bevorzugt ist n eine Zahl von 10-1000, besonders bevorzugt 10-500 und ganz besonders bevorzugt 10-100.

Bevorzugt ist ein Verfahren, welches dadurch gekennzeichnet ist, dass als Alkalihypohalogenit LiOCI, NaOCI, KOCI, LiOBr, NaOBr oder KOBr verwendet wird.

Besonders bevorzugt sind LiOCL, NaOCL und KOCI, ganz besonders bevorzugt ist NaOCI.

Das Oxidationsmittel wird bevorzugt als wässrige Lösung dem zu oxidierenden Alkohol zugesetzt. Die Konzentration kann in weitem Rahmen variieren, bevorzugt liegt sie bei 5-20 Gew.-% an aktivem Chlor, besonders bevorzugt bei 10-15 Gew.-%, bezogen auf den zu oxidierenden Alkohol.

Zusammen mit dem Oxidationsmittel kann die wässrige Lösung mit einer Base alkalisch gestellt werden. Bevorzugte Basen sind wässrige Lösungen von Alkali-oder Erdalkalihydroxiden, Alkali- oder Erdalkalicarbonaten bzw. die entsprechenden Hydrogencarbonate.

Besonders bevorzugt sind Alkalihydrogencarbonate, ganz besonders bevorzugt Natriumhydrogencarbonat.

Der pH-Wert der wässrigen Oxidationslösung ist nach Zugabe der gewünschten Base bevorzugt im Bereich 8-12, besonders bevorzugt im Bereich 9-11 und ganz besonders bevorzugt im Bereich 9-10.

Der zu oxidierende Alkohol kann flüssig oder fest sein. Bei flüssigen Alkoholen kann die Reaktion ohne Zusatz weiterer Lösungsmittel erfolgen, es kann jedoch zweckmässig sein, in stärkerer Verdünnung zu oxidieren. Feste Alkohole benötigen in jedem Fall ein geeignetes organisches Lösungsmittel.

Geeignete organische Lösungsmittel oder Lösungsmittelgemische sind solche, die mit Wasser nicht mischbar sind. Beispiele sind aliphatische Kohlenwasserstoffe, aromatische Kohlenwasserstoffel, chlorierte Kohlenwasserstoffe oder Gemische dieser Lösungsmittel mit Ketonen, Amiden oder Estern.

Bevorzugte Lösungsmittel sind aromatische Kohlenwasserstoffe oder Gemische davon mit Ketonen. Bevorzugte Beispiele sind Benzol, Toluol oder die Isomeren des Xylols, welche gegebenfalls mit Aceton gemischt sein können.

Das Mischungsverhältnis kann von 10:1 bis 2:1 betragen, bevorzugt ist 5:1 bis 2:1

Ganz besonders bevorzugt ist ein Gemisch Toluol/Aceton im Verhältnis 3:1.

Bevorzugt ist ein Verfahren bei welchem man ein an ein Merrifield-Polymer 4-oxy gebundenes 4-Oxy-2,2,6,6-tetramethylpiperidin-1-oxyl verwendet.

Sogenannte Merrifield-Polymere sind dem Fachmann bekannt und im Handel erhältlich. Es handelt sich dabei um chlormethyliertes Polystyrol, welches teilweise mit Divinylbenzol vernetzt und damit in üblichen organischen Lösungsmitteln unlöslich ist.

Der Vernetzungsgrad kann beispielsweise 1-5% betragen, typischerweise beträgt er 1-2%. Die Partikelgrösse kann in einem weiten Rahmen variieren, typischerweise beträgt sie 100-400 mesh. Der Chlorgehalt beträgt beispielsweise von 0,2 bis 5 mmol/g, gängige Polymere enthalten 0,6 bis 4mmol/g.

Schematisch lässt sich das Merrifield-Polymer und der Austausch des Chloratoms, wie nachstehend dargestellt, beschreiben.

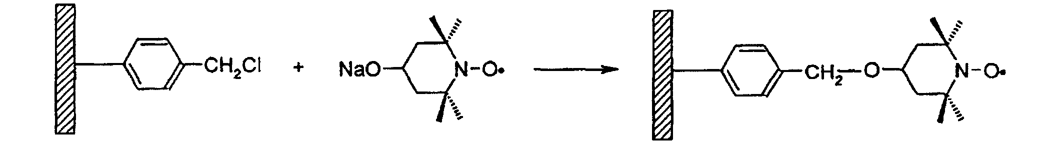

Bevorzugt setzt man den heterogenen Oxidationskatalysator in einer Menge von 0,1 bis 20 Gew-%, besonderes bevorzugt von 1 bis 10 Gew-% und ganz besonders bevorzugt von 2-6 Gew.-%, bezogen auf den eingesetzten Alkohol zu.

Bevorzugt ist ein Verfahren, in welchem man ein zweiphasiges Lösungsmittelsystem einsetzt, wobei eine Phase wässrig ist und das Oxidationsmittel enthält.

Geeignete Lösungsmittel bzw. Lösungsmittelgemische und ihre Bevorzugungen sind bereits vorstehend beschrieben.

Bevorzugt führt man die Reaktion bei einer Temperatur von weniger als 10° C durch.

Besonders bevorzugt ist ein Temperaturbereich von ca. 0° C bis 10° C.

Die Herstellung der Verbindungen der Formel (I)-(III) und die Herstellung des modifizierten Merrifield-Polymer erfolgen nach an sich bekannten Methoden gemäss nachstehendem Reaktionsschema.

In einem ersten Schritt wird die Verbindung 3 (4-Hydroxy-2,2,6,6-piperdin-1-oxyl (4-Hydroxy-TEMPO) mit Natriumhydrid umgesetzt. 4-Hydroxy-TEMPO selbst ist im Handel erhältlich.

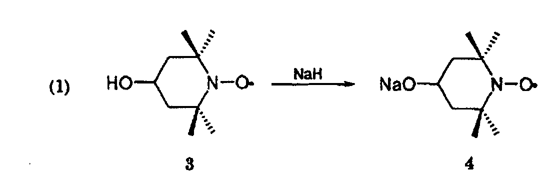

Entsprechend dem gewünschten Endprodukt setzt man die Verbindung 4 gemäss Reaktion 2, 3, 4 oder 5 weiter um.

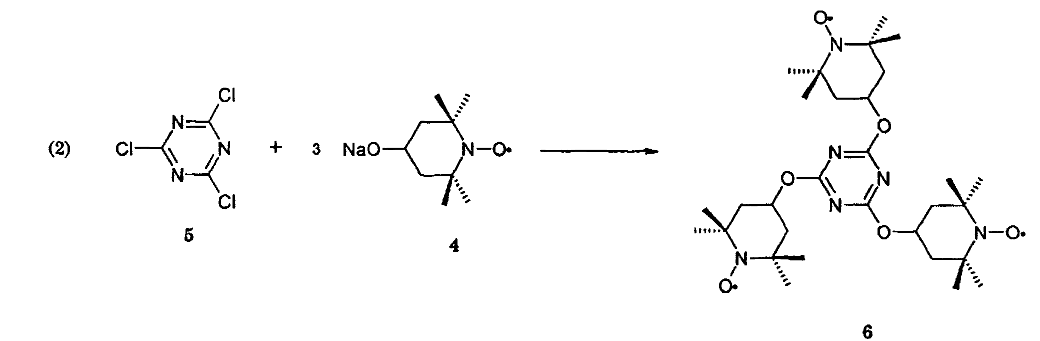

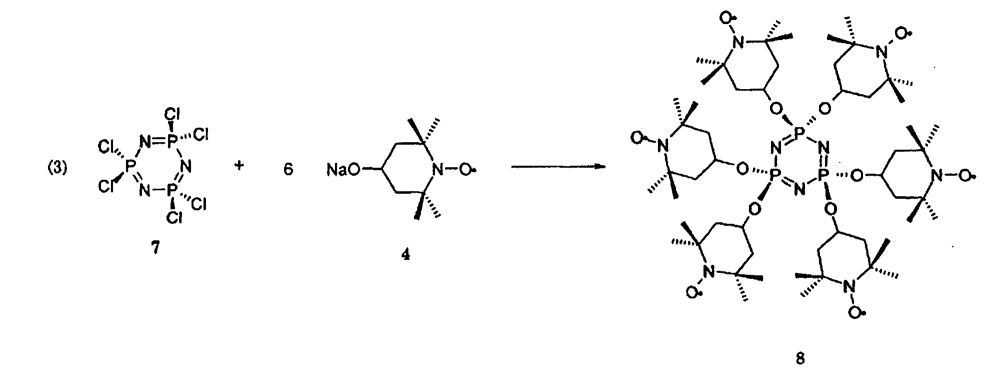

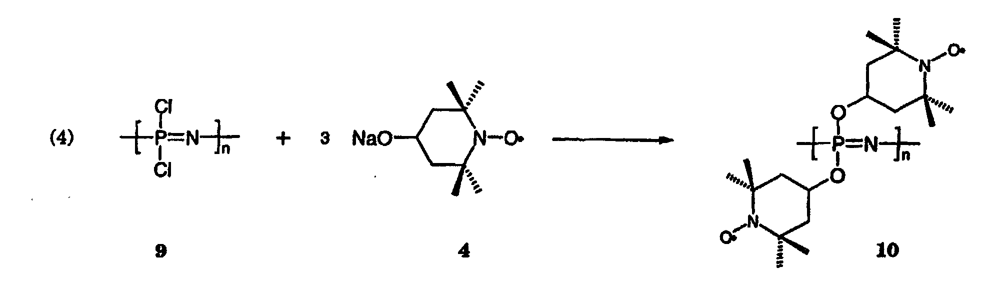

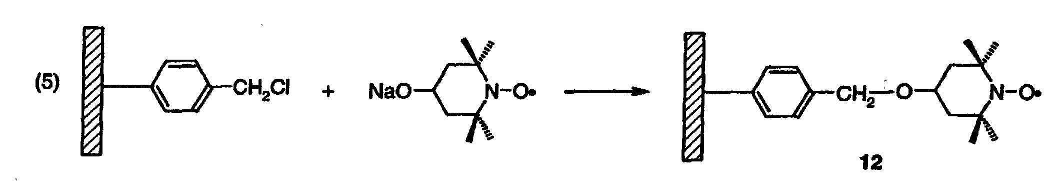

Die Verbindungen der Formel (III) sind neu und ebenfalls Gegenstand der Erfindung.

Ein weiterer Gegenstand der Erfindung ist die Verwendung einer Verbindung der Formel (I), (II), (III) oder ein an ein Merrifield-Polymer 4-oxy gebundenes 4-oxy-2,2,6,6-tetramethylpiperidin-1-oxyl als Katalysator für die selektive Oxidation von Alkoholen zu Ketonen oder Aldehyden mit einem Alkalihypohalogenit unter alkalischen Bedingungen.

Die nachstehenden Beispiele erläutern die Erfindung.

### Beispiele A: Herstellung der Oxidationskatalysatoren

### Beispiel A1

### Herstellung der Verbindung 6.

In einem 500 mL Schlenkgefäss werden 20g (113.6 mmol) 4-Hydroxy-TEMPO, 3 in 200 ml wasserfreiem Toluol gelöst und anschliessend 3.34 g (139.2 mmol) NaH in kleinen Portionen zugegeben. Der Reaktionsansatz wird bei Raumtemperatur ca. 12 h gerührt und anschliessend wird tropfenweise eine Lösung von 4.75 g (25.75 mmol) Cyanurchlorid 5 in ca. 50 ml Toluol zugegeben. Die Lösung wird zunächst bei Raumtemperatur 2.5 h und anschliessend 72 h bei ca. 70°-90°C gerührt. Nach Abkühlen auf Raumtemperatur wird die organische Phase dreimal mit jeweils 100 ml einer 10% wässrigen Lösung von Na₂CO₃ gewaschen und danach über Na₂SO₄ getrocknet. Alle flüchtigen Bestandteile werden bei 1.3·10⁻⁵ bars (10⁻² Torr) im Vakuum abgedampft und das verbleibende rote Öl aus wenig Essigsäureethylester umkristallisiert. Man erhält 9.3 g (61%) Produkt in Form feiner orange-roter Nadeln. Schmp. 164 - 166°C. Das Produkt ist unlöslich in H₂O aber löslich in CH₂Cl₂, CHCl₃, C₆H₅Cl, Toluol und Essigester.

### Beispiel A2

### Herstellung der Verbindung 8.

In einem 500 mL Schlenkgefäss werden 20g (113.6 mmol) 4-Hydroxy-TEMPO, 3 in 200 ml wasserfreiem THF gelöst und anschliessend 3.34 g (139.2 mmol) NaH in kleinen Portionen zugegeben. Der Reaktionsansatz wird bei Raumtemperatur ca. 12 h gerührt und anschliessend wird tropfenweise eine Lösung von 6.26 g (18 mmol) Hexachlorocyclotriphosphazen 7 in ca. 50 ml THF zugegeben und die Reaktionsmischung bei 70°C ca. 24 h unter Rückfluss erhitzt. Die flüchtigen Bestandteile werden nach Abkühlen auf Raumtemperatur im Vakuum ca. 1.3^{.}10⁻⁵ bars (ca. 10⁻² Torr) verdampft und der Rückstand in 100 ml CH₂Cl₂ gelöst. Die organische Phase wird zweimal mit je 50 ml einer 10% wässrigen Lösung von NaOH gewaschen und anschliessend je dreimal mit ca. 50 ml H₂O. Die organische Phase wird mit Na₂SO₄ getrocknet und die flüchtigen Bestandteile im Vakuum ca. 1.3^{.}10⁻⁵ bars (ca. 10⁻² Torr) abgedampft. Man erhält einen pulverförmigen orange-roten Festkörper, der in H₂O und Hexane unlöslich und in THF, CHCl₃ und CH₂Cl₂ relativ gut löslich ist.

### Beispiel A3

### Herstellung der Verbindung 10.

In einem 500 mL Schlenkgefäss werden 29.62 g (168.3 mmol) 4-Hydroxy-TEMPO, 3, in 200 mL THF gelöst und anschliessend 5.0 g (208 mmol) NaH zugegeben. Man lässt 12 h bei Raumtemperatur rühren und tropft anschliessend diese Reaktionsmischung in eine Lösung von 5g (43.1 mmol) Poly(dichlorophosphazen) 9 in 100 ml THF. Man lässt die Reaktionsmischung während 12 h bei Raumtemperatur rühren und erhitzt dann während zwei Stunden unter Rückfluss. Anschliessend wird die Reaktionsmischung auf ca. 10% des Volumens eingengt und auf Eiswasser gegeben. Das ausgefallene Polymer wird abfiltriert und erneut mit Eiswasser behandelt. Der lachsfarbene, pulverige Niederschlag wird abfiltriert, dann mit einem Gemisch aus THF und H₂O (20/80) und anschliessend mit Hexan gewaschen. Das entstandene Pulver wird während ca. 12 h im Vakuum getrocknet. Es werden 16 (95.7%) des Produkts 10 erhalten; Schmp. > 180°C. Verbindung 10 ist in CH₂Cl₂, CHCl₃, Aceton, THF und Toluol löslich, M_{w} ca. 25000.

### Beispiel A4

### Herstellung der Verbindung 12:

In einem 500 ml Schlenkgefäss werden 7g (40.63 mmol) 4-Hydroxy-TEMPO, 3 in 120 ml DMF oder THF (frisch destilliert) gelöst. Bei O° C werden zu der Lösung 1.6 g (66.67 mmol) NaH gegeben. Man lässt auf Raumtemperatur erwärmen und rührt 1 h nach. Anschliessend wird die Reaktionlösung im Eisbad auf 0° C gekühlt und 3.5 g Polymer (Merrifield-Polymer der Fa. Fluka, 200-400 mesh, 1% Divinylbenzol, 1,7mmol Cl/g) werden zugegeben. Man rührt 30 min. bei 0°C und lässt dann auf Raumtemperatur erwärmen. Der Ansatz wird 1 bis 4 Tage gerührt. Anschliessend wird mit Eiswasser verdünnt, aufgerührt und filtriert. Der Rückstand wird mit Eiswasser gewaschen, bis das Filtrat farblos ist. Anschliessend wird das Produkt in Toluol suspendiert und 1 bis 2 h gerührt, um nicht fixiertes 4-Hydroxy-TEMPO zu entfernen. Darauf wird nochmals filtriert und das gelbe Pulver im Luftstrom getrocknet. Es enthält 0,9 mmol N-Oxyl/g

### Beispiele B: Oxidation von Alkoholen

Allgemeine Vorschrift für die Oxidation eines primären oder sekundären Alkohols
a) Herstellung der NaOCI / NaHCO₃ Lösung:
   4 mL einer gesättigten Lösung von NaHCO₃ werden mit 2 mL einer NaOCl-Lösung (13-14%) gemischt. Die Lösung wird in einer verschlossenen Flasche bei 0°C gelagert.
b) Oxidationsvorschrift:
   In einen 250 ml Rundkolben werden 1.0 g des Alkohols und 0.25 g der Verbindung (12) aus Beispiel A4 gegeben und anschliessend in einem Gemisch aus 5 ml Aceton und 15 ml Toluol suspendiert. Bei Raumtemperatur wird der Ansatz 5-10 min stark gerührt, bis der Alkohol gelöst und das Harz aufgequollen ist. Das Reaktionsgefäss wird auf 0°C gekühlt. Unter starkem Rühren gibt man 6 ml der unter 1) hergestellten NaOCI / NaHCO₃-Lösung zu und lässt bei 0 - 5° C 0.5 h rühren. Nach der üblichen Aufarbeitung wird das Keton in nahezu quantitativer Ausbeute erhalten.
c) Rückgewinnung des Katalysators:
   Soll der Katalysator in mehreren unmittelbar aufeinanderfolgendenden Reaktionen eingesetzt werden, so wird er nach Ende der Umsetzung abfiltriert (G3-Fritte), kurz mit Aceton gewaschen und sofort wieder verwendet. Nach zehnmaligem Einsatz konnte kein Aktivitätsverlust festgestellt werden Die katalytische Aktivität der Verbindung (12) kann auch nach Lagerung aufrecht erhalten werden. Dazu wird der abfiltrierte Katalysator mehrmals mit Aceton/Toluol, dann mit Wasser, wiederum Aceton und schliesslich mit Aceton/Toluol gewaschen (Entfernung von NaOCI, Edukt und Produkt). Die Aufbewahrung erfolgt feucht in einem fest verschlossenen Behälter.

### Beispiel B1 und B2

### Oxidation einfacher Alkohole:

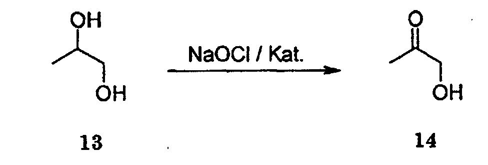

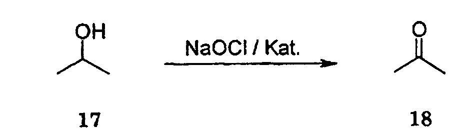

Die Versuche werden wie in der allgemeinen Vorschrift beschrieben durchgeführt. Dabei wird das α-Hydroxyketon 14 aus 13 bzw. Aceton 18 aus Isopropanol 17 in nahezu quantitativer Ausbeute erhalten.

### Beispiel B3

### Herstellung von α-Hydroxyacetophenon aus Phenylglykol:

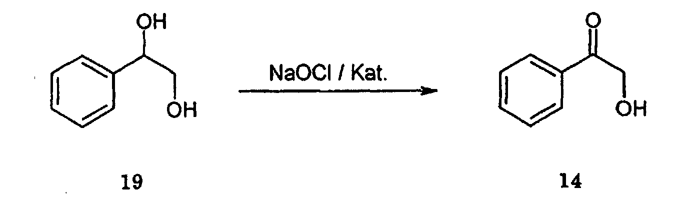

Phenylglykol 19 wird wie in der allgemeinen Vorschrift beschrieben mit NaOCI in Gegenwart von 5 Gewichtsprozent 12 als Katalysator zu α-Hydroxyacetophenon (20) umgesetzt. Das Produkt 20 wird in nahezu quantitativer Ausbeute als kristalliner Feststoff isoliert.

### Beispiel B4

### Herstellung von Acetylaceton:

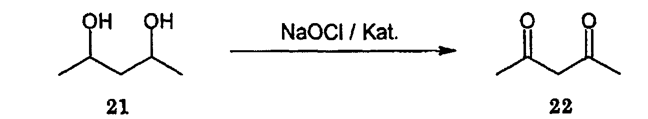

In einen 250 ml Rundkolben werden 1.0 g des Alkohols 21 und 0.25 g der Verbindung 12 vorgelegt, anschliessend 20 ml Toluol zugefügt. Das Reaktionsgefäss wird danach auf 0°C abgekühlt. Unter starkem Rühren versetzt man mit 12 ml der vorstehend beschriebenen NaOCI / NaHCO₃ und lässt bei 0-5°C ½ h rühren. Das 1,3-Diketon 22 wird in nahezu quantitativer Ausbeute als Reaktionsprodukt erhalten.

### Beispiel B5

### Herstellung von 2,2,4,6-Tetramethyl-1,3-dioxan (23):

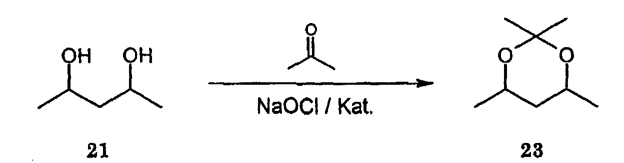

Die Reaktion wird wie in Beispiel 4 beschrieben durchgeführt, mit dem Unterschied, dass man die Reaktion statt mit 20 mL Toluol in einem Gemisch aus 15 ml Toluol und 5 ml Aceton durchführt. Als Reaktionsprodukt wird 2,2,4,6-Tetramethyl-1,3-dioxan (23) gaschromatographisch in nahezu quantitativer Ausbeute erhalten.

### Beispiel B6

### Herstellung von 2-Ethyl-2,4,6-trimethyl-1,3-dioxan (24):

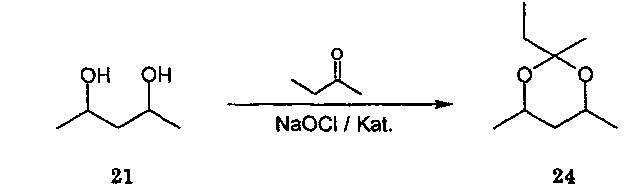

Die Reaktion wird wie Beispiel B4 beschrieben durchgeführt, mit dem Unterschied, dass anstatt von 20 ml Toluol ein Gemisch aus 15 ml Toluol und 5 ml 2-Butanon verwendet werden. 2-Ethyl-2,4,6-trimethyl-1,3-dioxan (24) wird in nahezu quantitativer Ausbeute als Reaktionsprodukt erhalten.

### Beispiel B7

### Herstellung von Tetrahydropyran-2-ol (21) und Tetrahydropyran-2-on (22)

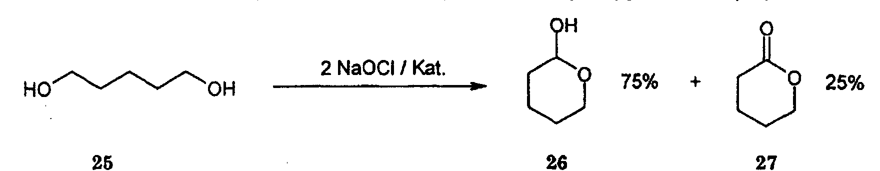

1,5-Pentandiol 25 wird wie in Beispiel B4 beschrieben mit basischer NaOCI in Gegenwart der Verbindung 12 als Katalysator oxidiert, mit dem Unterschied, dass das Oxidationsmittel Natriumhypochlorit (13% Aktiv-Chlor) stöchiometrisch eingesetzt wird. Nach 30 min Reaktionszeit wird ein Gemisch aus 75% 26 und 25% 27 erhalten.

### Beispiel B8

### Herstellung von Tetrahydropyran-2-on (27)

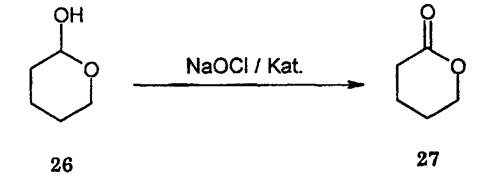

Das in Beispiel B7 hergestellte Gemisch aus 26 und 27 wird mit einem Überschuss einer auf pH 9.0-9.5 gepufferten Lösung von NaOCI wie beschrieben umgesetzt. Dabei wird 26 vollständig zu 27 oxidiert.

### Beispiel B9

### Herstellung von 2-(5-Hydroxy-pentoxy)-tetrahydropyran (26)

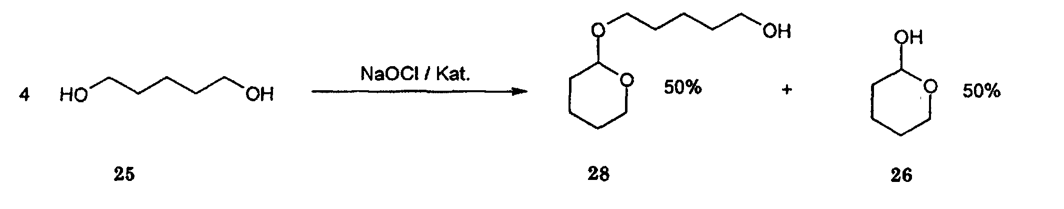

Die oben beschriebene Umsetzung wird mit einem vierfachen Überschuss des Diols 25 bezüglich NaOCI durchgeführt. Nach üblicher Aufarbeitung wird ein Gemsich aus 50% des Acetals 28 und 50% des Tetrahydropyran-2-ols 26 erhalten.

### Beispiel B10

### Herstellung von Benzil (29) aus Benzoin:

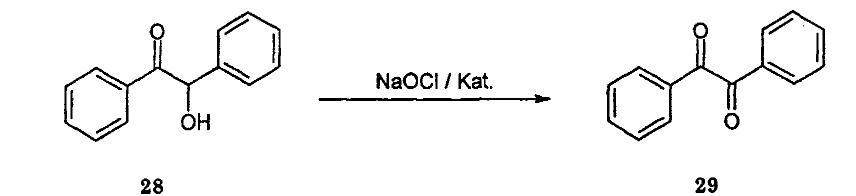

5.0 g (23.5 mMol) Benzoin (28) werden wie in der allgemeinen Vorschrift beschrieben mit Natriumhypochlorit in Gegenwart von 1 g der Verbindung 12 bei 0 - 5° C in 50 mL Toluol / Aceton 3:1 umgesetzt. Während der Hypochlorit-Zugabe geht der im Reaktionsgemisch zunächst vorliegende Feststoff in Lösung. Nach erfolgter Zugabe lässt man noch 45 min nachrühren. Kontrolle mit Dünnschichtchromatographie zeigt vollständigen und selektiven Umsatz an. Nach der üblichen Aufarbeitung und anschliessender Umkristallisation des Rohproduktes aus n-Hexan werden 4.6 g Benzil (29) (93% d. Th.) erhalten.

## Patentansprüche

1. Verfahren zur selektiven Oxidation von Alkoholen zu Ketonen oder Aldehyden mit einem Alkalihypohalogenit unter alkalischen Bedingungen, **dadurch gekennzeichnet, dass** man die Oxidation in Gegenwart eines im Reaktionsmedium unlöslichen heterogenen Oxidationskatalysators, ausgewählt aus der Gruppe enthaltend die Verbindungen der Formel (I), (II), (III) 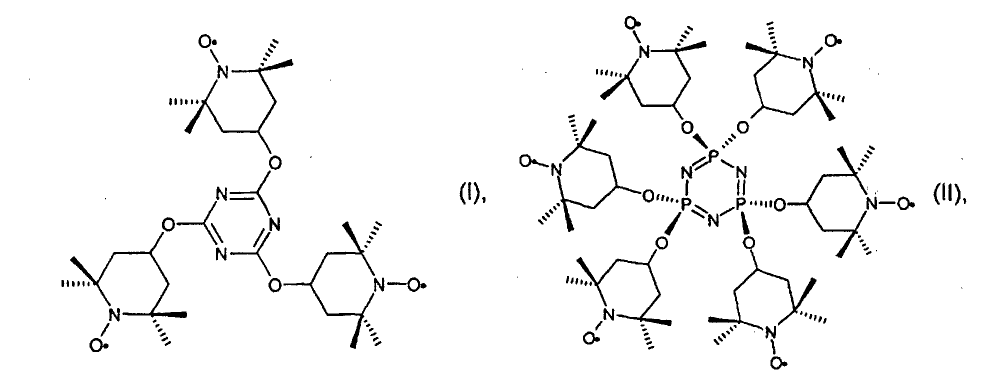 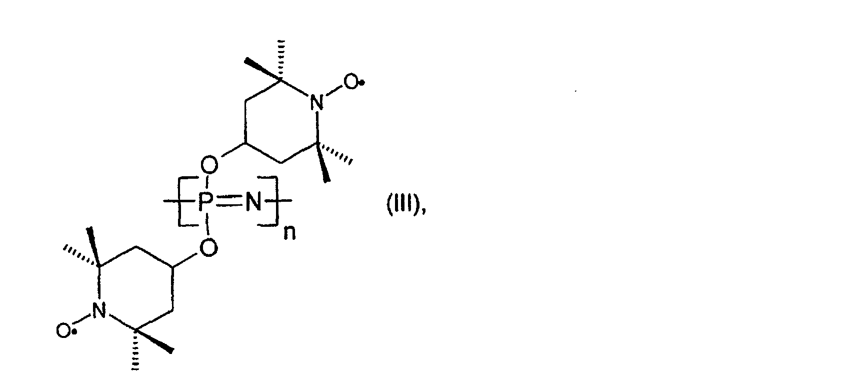 worin n eine Zahl von 3-3000 ist; oder
ein an ein Merrifield-Polymer 4-oxy gebundenes 4-Oxy-2,2,6,6-tetramethylpiperidin-1-oxyl, durchführt.

2. Verfahren nach Anspruch 1, **dadurch gekennzeichnet, dass** als Alkalihypohalogenit LiOCI, NaOCI, KOCI, LiOBr, NaOBr oder KOBr verwendet wird.

3. Verfahren nach Anspruch 1, **dadurch gekennzeichnet, dass** man ein an ein Merrifield-Polymer 4-oxy gebundenes 4-Oxy-2,2,6,6-tetramethylpiperidin-1-oxyl verwendet.

4. Verfahren nach Anspruch 1, **dadurch gekennzeichnet, dass** man den heterogenen Oxidationskatalysator in einer Menge von 0,1 bis 20 Gew-%, bezogen auf den eingesetzten Alkohol zusetzt.

5. Verfahren nach Anspruch 1, **dadurch gekennzeichnet, dass** man ein zweiphasiges Lösungsmittelsystem einsetzt, wobei eine Phase wässrig ist und das Oxidationsmittel enthält.

6. Verfahren nach Anspruch 1, **dadurch gekennzeichnet, dass** man die Reaktion bei einer Temperatur von weniger als 10° C durchführt.

7. Verbindungen der Formel III nach Anspruch 1.

8. Verwendung einer Verbindung der Formel (I), (II), (III) oder ein an ein Merrifield-Polymer 4-oxy gebundenes 4-oxy-2,2,6,6-tetramethylpiperidin-1-oxyl als Katalysator für die selektive Oxidation von Alkoholen zu Ketonen oder Aldehyden mit einem Alkalihypohalogenit unter alkalischen Bedingungen.

## Claims

1. A process for the selective oxidation of alcohols to ketones or to aldehydes by means of an alkali hypohalite under alkaline conditions, which comprises carrying out the oxidation in the presence of a heterogeneous oxidation catalyst that is insoluble in the reaction medium and is selected from the group comprising the compounds of formula (I), (II), (III) 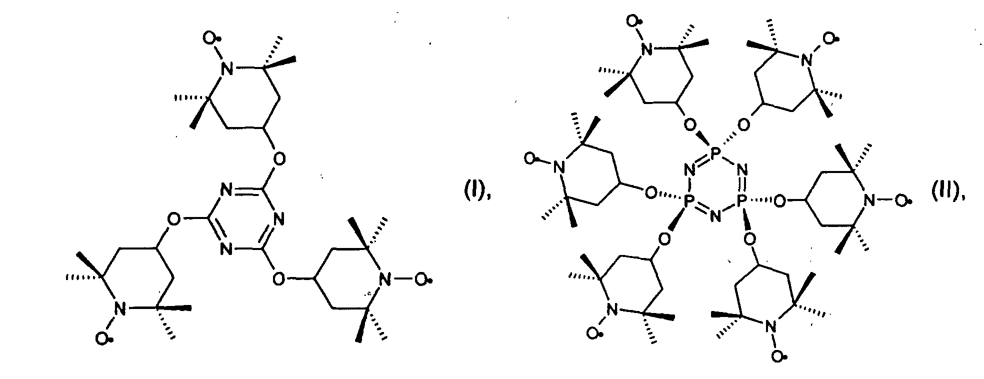 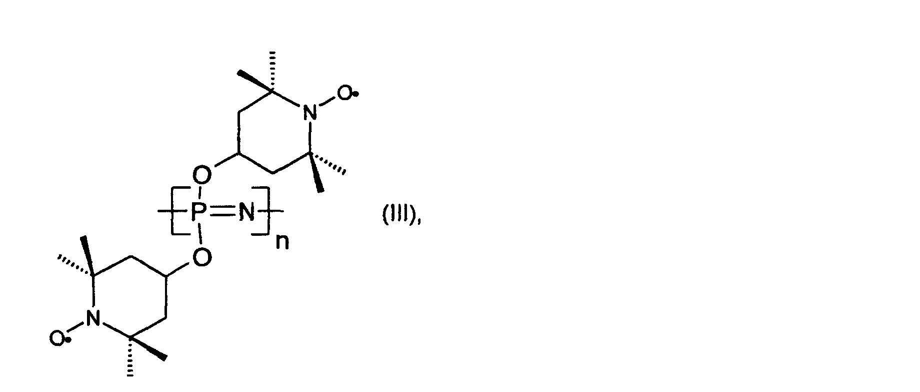 wherein n is a number from 3 to 3000; or
a 4-oxy-2,2,6,6-tetramethylpiperidin-1-oxyl that is 4-oxy-bound to a Merrifield polymer.

2. A process according to claim 1, which comprises using as alkali hypohalite LiOCI, NaOCl, KOCI, LiOBr, NaOBr or KOBr.

3. A process according to claim 1, which comprises using a 4-oxy-2,2,6,6-tetramethylpiperidin-1-oxyl that is 4-oxy-bound to a Merrifield polymer.

4. A process according to claim 1, which comprises adding the heterogeneous oxidation catalyst in an amount of from 0.1 to 20 % by weight, based on the alcohol used.

5. A process according to claim 1, which comprises using a two-phase solvent system in which one phase is aqueous and comprises the oxidising agent.

6. A process according to claim 1, which comprises carrying out the reaction at a temperature of less than 10°C.

7. A compound of formula III according to claim 1.

8. The use of a compound of formula (I), (II), (III), or a 4-oxy-2,2,6,6-tetramethylpiperidin-1-oxyl 4-oxy-bound to a Merrifield polymer, as a catalyst for the selective oxidation of alcohols to ketones or to aldehydes by means of an alkali hypohalite under alkaline conditions.

## Revendications

1. Procédé pour l'oxydation sélective d'alcools en cétones ou aldéhydes par un hypohalogénure alcalin sous conditions alcalines, **caractérisé en ce qu'**on met en oeuvre l'oxydation en présence d'un catalyseur d'oxydation hétérogène insoluble dans le milieu réactionnel, pris dans le groupe comprenant les composés de formule (I), (II), (III) 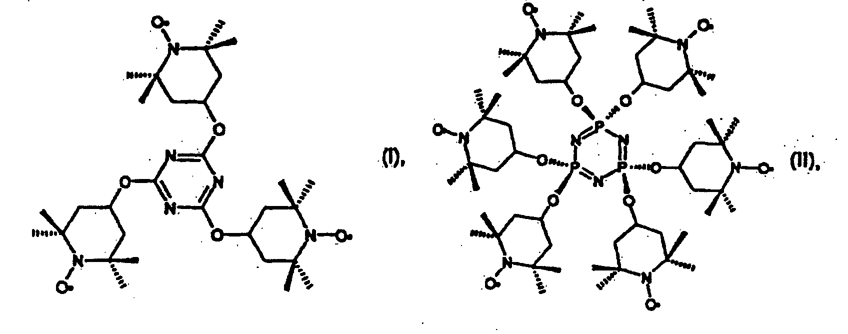 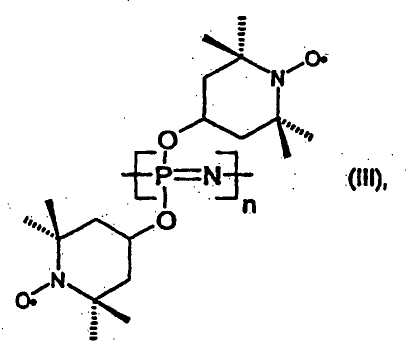 où n est un nombre de 3 à 3000 ; ou un 4-oxy-2,2,6,6-tétraméthylpipéridin-1-oxyle lié par 4-oxy à un polymère de Merrifield.

2. Procédé selon la revendication 1, **caractérisé en ce qu'**on utilise en tant qu'hypohalogénite alcalin LiOCl, NaOCl, KOCl, LiOBr ou KOBr.

3. Procédé selon la revendication 1, **caractérisé en ce qu'**on utilise un 4-oxy-2,2,6,6-tétraméthylpipéridin-1-oxyle lié par 4-oxy à un polymère de Merrifield.

4. Procédé selon la revendication 1, **caractérisé en ce qu'**on ajoute le catalyseur hétérogène dans une quantité de 0,1 à 20 % en poids, par rapport à l'alcool utilisé.

5. Procédé selon la revendication 1, **caractérisé en ce qu'**on utilise un système de solvants biphasique, une phase étant aqueuse et renferme l'agent oxydant.

6. Procédé selon la revendication 1, **caractérisé en ce qu'**on met en oeuvre la réaction à une température inférieure à 10°C.

7. Composés de formule III selon la revendication 1.

8. Utilisation d'un composé de formule (I), (II), (III) ou d'un 4-oxy-2,2,6,6-tétraméthylpipéridin-1-oxyle lié par 4-oxy à un polymère de Merrifield, en tant que catalyseur pour l'oxydation sélective d'alcools en cétones ou aldéhydes par un hypohalogénite alcalin sous conditions alcalines.
